# EUROPÄISCHE PATENTSCHRIFT

(11) **EP 4 321 132 B1**
(45) Veröffentlichungstag und Bekanntmachung des Hinweises auf die Patenterteilung: **24.07.2024**
(21) Anmeldenummer: 23180536.7
(22) Anmeldetag: 21.06.2023
(51) Int. Cl.: A61F 2/18, A61F 5/08

(54) **VERBESSERTES SEPTUM-IMPLANTAT MIT EINEM ZENTRALEN RÜCKENABSCHNITT UND DREI TEILABSCHNITTEN**
IMPROVED SEPTUM IMPLANT WITH A CENTRAL BACK PORTION AND THREE PARTIAL PORTIONS
IMPLANT SEPTALE AMELIORE COMPRENANT UNE PARTIE DORSALE CENTRALE ET TROIS PARTIES

(30) Priorität: 10.08.2022 DE 102022120193
(43) Veröffentlichungstag der Anmeldung: 14.02.2024
(73) Patentinhaber: Heinz Kurz GmbH, 72144 Dusslingen (DE)
(72) Erfinder: STIEGELE, Thomas, 72585 Riederich (DE); SANDER, Alexander, 79822 Titisee-Neustadt (DE); MERTENS, Matthias, 24568 Kaltenkirchen (DE)
(74) Vertreter: Kohler Schmid Möbus Patentanwälte

(56) Entgegenhaltungen:
- EP-B1- 1 475 056
- DE-B3- 102006 023 058
- DE-B4- 102012 107 123
- US-A- 5 716 405
- US-A1- 2013 317 540
- US-B1- 6 322 590

## Beschreibung

Die Erfindung betrifft ein rhinologisches Implantat zur Begradigung der Nasenscheidewand, das beiderseits des Septums der menschlichen Nase auf der jeweiligen Außenfläche des Septums in der jeweils linken und rechten Seite der Nasenhöhle befestigbar ist, wobei das Implantat einen zentralen Rückenabschnitt aufweist, der flach oder nur sehr leicht von einer Horizontalebene nach oben mit einem Spreizungswinkel ω > 100° abgewinkelt oder tonnenförmig mit einem Krümmungsradius r ≥ 0,5mm von der Horizontalebene nach oben gekrümmt ist und im implantierten Zustand die freie Unterkante des Septums umgreift, wobei zwei Seitenabschnitte des Implantats beiderseits des zentralen Rückenabschnitts vorgesehen sind, die symmetrisch zum zentralen Rückenabschnitt unter einem Winkel ϕ von jeweils etwa 90° gegen den zentralen Rückenabschnitt nach oben abgekantet verlaufen, und die im implantierten Zustand auf den beiden gegenüberliegenden Außenflächen des Septums vollflächig anliegen, wobei das Implantat vor der Implantation aus einem zunächst flachen Zuschnitt in seine spätere, implantierfähige Raumform aufgefaltet ist, wobei die beiden Seitenabschnitte des Implantats jeweils einen ersten Teilabschnitt aufweisen, der sich unmittelbar an den zentralen Rückenabschnitt anschließt und im Wesentlichen parallel zu diesem verläuft, und wobei die beiden Seitenabschnitte jeweils einen zweiten Teilabschnitt aufweisen, der sich an den jeweiligen ersten Teilabschnitt anschließt und gegenüber diesem abgewinkelt verläuft.

Ein derartiges rhinologisches Implantat zur Begradigung der Nasenscheidewand geht hervor aus dem generischen Stand der Technik DE 10 2012 107 123 B4 :zi EP 2 692 313 B1 ≈ US 9,895,252 B2 (= Referenz [1]) der Anmelderin.

### Hintergrund der Erfindung

Generell sind Implantate zur Septum-Begradigung beispielsweise beschrieben auf der Internetseite vom 09.08.2022 https://www.bess.eu/de/rhinoloqie/septumschienen/ (= Referenz [2]).

Die Einpflanzung eines Implantats, d. h. eines in der Regel körperfremden Gewebsstücks oder Stoffes, in den menschlichen Körper ist in der Medizintechnik ein lange bekanntes Verfahren, das in vielen Variationen zur Behebung funktioneller Störungen verschiedener Körperteile und/oder psychischer Beeinträchtigungen vorgenommen wird.

Bekanntlich besteht der knorpelige Anteil der menschlichen Nase aus der Nasenscheidewand (= Septum), dem Dreiecksknorpel und dem Flügelknorpel. Die vorliegende Erfindung beschäftigt sich mit der Begradigung des Septums. In vielen Fällen der funktionellen, aber auch der kosmetischen Nasenchirurgie ist es das Ziel, das Septum zu begradigen, um den Luftstrom innerhalb des Nasenverlaufs zu verbessern oder schlicht die Nase kosmetisch zu verschönern. Meist geht dieser Vorgang noch mit der Veränderung anderer Strukturen einher. Derzeit werden zur Begradigung des Septums unterschiedliche Naht- oder Knorpeltransplantattechniken verwendet.

Im Folgenden sollen zunächst die Unterschiede zwischen einer Septumplastik und der Columella Strut Technik erläutert werden:
Die Septumplastik zielt ausschließlich darauf ab, das Septum zu begradigen und damit die Luftzufuhr über die Nase zu optimieren. Es handelt sich also um rein funktionelle Chirurgie. Hierzu gibt es eine Reihe von chirurgischen Techniken, die über Nähte und Transplantate zum Ziel führen sollen.

Die Begradigung eines deviierten Septums kann eine der größten Herausforderungen bei einer Rhinoplastik sein. Unterschiedliche und im Wesentlichen von der Erfahrung und dem Geschick des Operateurs abhängige Verfahren kommen hierfür in Betracht.

Disartikulationstechniken bis hin zu gezielten Knorpelinzisionen können mehr oder weniger eine Rolle spielen. In einer Sache ist sich die Fachwelt hier einig, dass eine Septumbegradigung gerade im mittleren Gewölbe eine schwierige Angelegenheit und keineswegs eine einfache Operation ist.

Im Gegensatz zur Septumbegradigung zielt die Columella Strut Technik darauf ab, die Nasenspitze aus kosmetischen Gründen heraus zu erhöhen. Dabei wird ein Steg zwischen die beiden Enden der beiden Flügelknorpel geschoben, um die Spitze der Nase zu erhöhen. Das Transplantat/ Implantat wird in eine Tasche zwischen den medialen Schenkeln der Flügelknorpel über die Spina nasalis anterior aufgestellt und zwischen den medialen Schenkeln mit durchgreifenden Nähten befestigt.

Dies ist beispielsweise beschrieben in der US 2012/0078367 A1 (= Referenz [3]). Hier wird von einem Implantat gesprochen, das biologisch abgebaut wird und somit nicht für den Langzeitverbleib vorgesehen ist. Die Zielregion ist das untere Drittel der Nase, hauptsächlich die Region um die Nasenspitze. Zwar ist hier auch die Rede von einer Anbindung des Implantates an das Septum, allerdings nicht zur Begradigung desselben, sondern zur Verlängerung, falls durch äußere Einwirkung oder aus kosmetischer Sicht das Septum verkürzt ist. Die gebogenen Teile des beschriebenen Implantats sind in ihrer Längenausdehnung höchstens etwa 20 mm lang, d. h. sie reichen, wenn man sie auf die Basis der Spina Nasalis setzt und die beiden Schenkel der Flügelknorpel aufnimmt, gerade noch ans Ende des Septums. Letzteres wird damit also niemals begradigt. Bereits der Sitz des Implantats im implantierten Zustand wäre für Septumbegradigung völlig ungeeignet.

Allerdings ist das Implantat nach Referenz [3] keinesfalls zur Begradigung der Nasenscheidewand geeignet, und ist auch nicht beiderseits des Septums der menschlichen Nase auf der jeweiligen Außenfläche des Septums in der jeweils linken und rechten Seite der Nasenhöhle befestigbar.

Vielmehr handelt es sich bei dem Implantat gemäß Referenz [3] um ein "Columella Strut", nicht jedoch um eine gattungsgemäße Septumplastik entsprechend der vorliegenden Erfindung. Ein Hilfsmittel zur Begradigung des Septums thematisiert und erwähnt Referenz [3] überhaupt nicht.

Auch in der WO 2008/153263 A1 (= Referenz [4]) werden ausschließlich Techniken beschrieben, die zum Ziel haben, die Nasenspitze anzuheben. So ist dort zwar ebenfalls vom Septum die Rede. Beschrieben wird etwa die Technik, dass das Implantat an der oberen Grenze des Septumknorpels angebracht werden muss. Allerdings geschieht dies auch hier wieder lediglich zur Stabilisierung des Nasenspitzenimplantats.

Die bekannten Implantations-Systeme nach Referenz [3] und nach Referenz [4] versuchen also, das Ende des Septums zur Stabilisierung oder zur Verlängerung zu verwenden. Keines der beiden Systeme zielt jedoch darauf ab oder wäre geeignet dazu, das Septum dauerhaft zu begradigen.

Implantate zur Spreizung der Nasenflügel sind etwa beschrieben in US 6,322,590 B1 (= Referenz [5]) oder in EP 1 475 056 B1 (= Referenz [6]) oder in DE 2006 023 058 B3 (= Referenz [7]). Auch diese Implantations-Systeme sind keinesfalls zur Septum-Begradigung ausgebildet oder geeignet.

Zur Begradigung des Septums hingegen sind Techniken mit PDS-Folien bekannt (etwa beschrieben in: HNO.1999 Jun; 47 (6): 546-50 (= Referenz [8])), welche zurechtgeschnitten und auf das Septum aufgenäht werden. Die hauchdünnen Folien-Implantate resorbieren nach 10 bis 25 Wochen im Wesentlichen rückstandsfrei.

Stabilere Implantate zur Septumbegradigung stellen die Septumschienen nach Reuter aus Silikon dar, wie sie auf der eingangs zitierten Internetseite der Firma Bess beschrieben sind. Diese Implantate dienen zur Schienung des Septums und zur Minimierung des Adhäsionsrisikos zwischen Septum und lateraler Nasenwand nach Rhinoplastik. Sie sind zum leichteren Einführen und Entfernen geschlitzt und weisen vorgestanzte Löcher zur Nahtbefestigung auf. Als Material wird Fluorplastik angegeben. Die Schienen müssen allerdings nach ein paar Wochen wieder aus der Nase herausgenommen werden, da Silikon als nicht langzeit-stabil gilt und potentiell zu Infektionen führt.

CN 21 538 4901 U (= Referenz [9]) offenbart eine Nasenkorrekturstütze für die plastische Chirurgie, die einen Columella-nasi-Stützabschnitt, einen Nasenseptum-Verlängerungsabschnitt, Blättchen des Columella-nasi-Stützabschnitts, Blättchen des Nasenseptum-Verlängerungs-abschnitts, eine Nasenspitzecke und eine Columella-nasi umfasst mit einer Klemmnut am Stützende, einer Klemmnut am Kopfende der Nasenscheidewandverlängerung und einer U-förmigen Nut.

US 11,241,306 B2 (= Referenz [10]) beschreibt Nasenimplantate mit einem planaren Profil, welches teilweise offene Räume aufweist. Ein solches Nasenimplantat kann kompressibel entlang einer oder mehrerer Dimensionen sein, wie etwa der Breite oder der Länge des planaren Profils.

Gegenüber dem in den Referenzen [2] bis [10] gezeigten Stand der Technik vermeidet der eingangs zitierte Stand der Technik gemäß Referenz [1] die Nachteile der Septumschienen nach Reuter und das Implantat liegt nach der Operation dauerhaft eng an der Nasenscheidewand an.

Die Grundidee von Referenz [1] ist es, das Septum einfach mit einer speziell geformten und dadurch mechanisch dauerhaft stabilen Struktur zu begradigen. Dabei ist es wichtig, dass das Implantat gemäß Referenz [1] geometrisch so ausgebildet ist, dass der gesamte Bereich des Septums abgedeckt wird. Dies wird dadurch erreicht, dass das Implantat die freie Unterkante des Septums umgreift. Weiter sind beide Seiten des Implantats so perforiert, so dass das Implantat einfach und sicher an beiden Seiten des Septums fixiert werden kann. Die Dicke und damit einhergehende Steifigkeit des Implantats ist insofern wichtig, als dass der Septumknorpel sich langfristig der Implantatform angleichen sollte und nicht umgekehrt.

Die beiden Seitenabschnitte des Implantats gemäß Referenz [1] weisen jeweils einen ersten Teilabschnitt auf, der sich unmittelbar an den zentralen Rückenabschnitt anschließt und im Wesentlichen parallel zu diesem verläuft, und die beiden Seitenabschnitte weisen zu ihren freien Enden hin jeweils einen zweiten Teilabschnitt auf, der sich an den jeweiligen ersten Teilabschnitt anschließt und gegenüber diesem abgewinkelt verläuft. Durch diese einfachen Maßnahmen wird die mechanische Langzeit-Stabilität des Implantats erhöht. Außerdem wird der flächige feste Sitz des Implantats an den beiden Seitenflächen der Nasenscheidewand verbessert.

Eine vergleichbare geometrische Vorschrift im Stand der Technik gemäß den Referenzen [2] bis [10] liegt nicht im Funktionsumfang der dort beschriebenen Columella-Strut-Technik sowie der Nasenflügel-Spreizung und bedient daher ein ganz anderes, eigenständiges Versorgungsfeld.

### Aufgabe der Erfindung

Der vorliegenden Erfindung liegt demgegenüber die Aufgabe zugrunde, mit unaufwändigen technischen Mitteln ein Implantat der gattungsbildenden Art gemäß Referenz [1] mit den eingangs definierten Merkmalen dahingehend zu modifizieren, dass eine noch bessere mechanische Langzeit-Stabilisierung des Septums ermöglicht und der flächige feste Sitz des Implantats an den beiden Seitenflächen der Nasenscheidewand noch weiter verbessert wird. Insbesondere soll eine möglichst gleichmäßige Verteilung der Anlagekräfte bei dem auf dem Septum angebrachten Implantat erreicht werden.

### Kurze Beschreibung der Erfindung

Diese Aufgabe wird auf überraschend einfach und kostengünstig zu realisierende Weise dadurch gelöst, dass sich an einem dem jeweiligen zweiten Teilabschnitt gegenüberliegenden Ende des jeweiligen ersten Teilabschnitts jeweils ein dritter Teilabschnitt anschließt und gegenüber dem jeweiligen ersten Teilabschnitt ebenfalls abgewinkelt verläuft.

Die neue, erfindungsgemäße Grundidee ist es, das Implantat in der Längsrichtung des zentralen Rückenabschnitts und des daran unmittelbar anhängenden ersten Teilabschnitts des jeweiligen Seitenabschnitts symmetrischer zu gestalten. Insbesondere werden durch den neu vorgesehenen dritten Teilabschnitt die Anlagekräfte des Implantats gegenüber dem Septum gleichmäßiger auf die gesamte Anlagefläche verteilt. Ein Kippmoment, welches bei nur einem einseitig vorhandenen zweiten Teilabschnitt gegebenenfalls auftreten kann, wird durch die beiderseitige Positionierung von jeweils zweiten und dritten Teilabschnitten bezüglich der ersten Teilabschnitte von vornherein kompensiert, idealerweise sogar gänzlich ausgeschlossen.

Damit wird ohne größeren Fertigungsaufwand eine besonders gute geometrische Anpassung des Implantats an die (normale) Ausgestaltung des menschlichen Septums und eine besonders gute Anlage des Implantats an den Außenflächen desselben erreicht. Insbesondere entstehen nach der Operation keine Hohlräume zwischen dem Implantat und dem Septum, weder im Bereich des zentralen Rückenabschnitts noch an den beiderseits daran anschließenden Seitenabschnitten. Vielmehr liegt das Implantat über seine gesamte dem Septum zugewandte Oberfläche eng an diesem an, was auch einen besonders guten mechanischen Halt des Implantats am Septum bewirkt.

Bei den meisten aktuellen Septum-Implantaten zur Korrektur von Septum-Deviationen oder Perforationen des Septums werden die Implantat-Strukturen mit Knorpel gestützt.

Demgegenüber haben die Implantate gemäß Referenz [1] sowie nach der vorliegenden Erfindung vielerlei Vorteile:
Es muss kein Knorpel aus anderen Bereichen (normalerweise Rippe) entnommen werden. Dadurch verringert sich die Operationszeit und es besteht weniger Infektionsgefahr.

Durch vorkonfektionierte erfindungsgemäße Implantate ist der Eingriff reproduzierbar, standardisiert und planbar.

Es ist zu erwarten, dass eine Stabilisierung aus Metall deutlich länger die gewünschte Begradigung leistet als dies mit verformbarem Knochen möglich wäre.

Weiter ist zu erwarten, dass das erfindungsgemäße Implantat deutlich dünner aufträgt als vergleichbar stabiler Knorpel, was sich positiv auf den gewünschten Luftdurchsatz auswirkt.

### Bevorzugte Ausführungsformen der Erfindung

Ganz besonders bevorzugt sind Ausführungsformen des erfindungsgemäßen Implantats, welche sich dadurch auszeichnen, dass der dritte Teilabschnitt eine größere vom ersten Teilabschnitt wegragende Längenausdehnung aufweist als der zweite Teilabschnitt.

Damit wird eine an die Anatomie des Septums besonders gut angepasste Geometrie des Implantats erreicht.

In einer weiteren bevorzugten Ausführungsform der Erfindung weisen die beiden Seitenabschnitte des Implantats jeweils eine Bumerang-förmige Kontur auf.

Für die Form des Implantats gibt es mehrere Möglichkeiten. In einer einfachen Ausführung kann das Implantat eine winkelförmige Kontur aufweisen, insbesondere in der Ebene V-förmig gebogen sein, wobei zweckmäßigerweise die Spitze des "V" etwas abgerundet sein sollte, um Verletzungen zu vermeiden. Das Implantat kann auch trapezförmig gestaltet sein und/oder kompliziertere Strukturen mit Verzweigungen aufweisen, die nach dem Aufbiegen des Implantats in seine räumliche Endform zu einer Stabilisierung beitragen können.

Bei vorteilhaften Varianten der Erfindung sind die Abkantungswinkel der freien Enden der beiden Seitenabschnitte so gestaltet, dass sich die Seitenabschnitte im implantierten Zustand in einem engen räumlichen Kontakt, insbesondere in einer beiderseits unter Spannung stehenden, vorzugsweise symmetrischen Verklemmung mit dem Septum befinden, was zu einem besonders guten und dauerhaften Sitz des Implantats beiträgt.

Bei weiteren vorteilhaften Varianten ist der Rückenabschnitt aus Stegen einer Netzstruktur aufgebaut, um dem Implantat in diesem Bereich eine erhöhte Flexibilität durch Dehnbarkeit und Stauchbarkeit zu verleihen. Ergänzend oder alternativ können bei weiteren Ausführungsformen auch in die Seitenabschnitte Stege einer Netzstruktur eingebaut sein, was die flächenmäßige Flexibilität noch weiter erhöht.

Möglich sind auch Varianten des erfindungsgemäßen Implantats, die sich dadurch auszeichnen, dass in die Seitenabschnitte ein oder mehrere Spikes eingearbeitet sind. Letztere verkrallen sich bei der Implantation im Septum-Knorpel und sorgen so für einen exzellenten und äußerst dauerhaften Halt des Implantats.

In einer Klasse von besonders bevorzugten Ausführungsformen kann das Implantat Perforationen aufweisen. Damit wird einerseits das Gewicht des Implantats verringert, andererseits der Anteil von körperfremdem Material, das durch das Implantat in den menschlichen Körper eingebracht wird, soweit wie möglich reduziert. Außerdem fördern die Perforationen das Verwachsen des Implantats mit dem Gewebe.

Die Perforationen sind vorzugsweise sowohl an den Seitenabschnitten des Implantats als auch an dem dazwischenliegenden Rückenabschnitt vorhanden und zum Beispiel als kreisrunde Löcher oder als Langlöcher ausgebildet.

Weiter dienen die Perforationen einer sicheren Fixierung am Dreiecksknorpel mittels einer Naht.

Damit kann zudem sichergestellt werden, dass das Knorpelgewebe des Septums ausreichend mit Nährstoffen versorgt wird.

Die Herstellung einer dauer-haltbaren Befestigung des Implantats durch Aufnähen am Knorpel ist allerdings in der Regel zeitintensiv und manchmal auch aufgrund der räumlichen Verhältnisse etwas kompliziert. Bei vorteilhaften Varianten der Erfindung ist daher ein im implantierten Zustand des Implantats das Septum vollständig durchquerendes, die beiden bezüglich des Septums gegenüberliegenden Seitenabschnitte miteinander verbindendes Verbindungselement, vorzugsweise ein Nietelement, vorhanden, mit welchem das Implantat über eine einfach herzustellende Perforation durch das Septum dauerhaft und sicher befestigt werden kann. Diese Art der Befestigung kann freilich auch mit den anderen, oben beschriebenen Befestigungsarten kombiniert werden, um einen besonders gesicherten Sitz des Implantats am Septum zu garantieren.

Ganz besonders bevorzugt sind Weiterbildungen dieser Ausführungsformen, bei welchen die Perforationen zusammen einen größeren Flächeninhalt aufweisen als die die Perforationen umgebenden, insbesondere Stegförmig ausgebildeten, festen Teilabschnitte der Seitenabschnitte.

Damit lassen sich die Masse und mithin das Gewicht des Implantats deutlich minimieren.

Das Knorpelgewebe des Septums kann dadurch besonders gut mit Nährstoffen versorgt werden.

Für spezielle Anwendungen der Erfindung kann es günstig sein, wenn zumindest ein Teil der Perforationen Polyederförmig oder Wabenförmig ausgebildet ist.

Dies ermöglicht eine hohe Stabilität auch bei geringem Materialeinsatz.

Bevorzugt ist eine Klasse von Ausführungsformen der Erfindung, bei welchen der zentrale Rückenabschnitt mit Perforationen versehen ist, und die Perforationen des zentralen Rückenabschnitts zusammen einen kleineren Flächeninhalt aufweisen als die die Perforationen umgebenden festen Teilabschnitte des zentralen Rückenabschnitts.

Damit lassen sich die Masse und mithin das Gewicht des Implantats deutlich minimieren. Außerdem wird hierdurch wiederum eine ausreichende Versorgung des Knorpelgewebes am Septums mit Nährstoffen sichergestellt.

Vorteilhafte Weiterbildungen dieser Klasse von Ausführungsformen zeichnen sich dadurch aus, dass die Perforationen Schlitzförmig, vorzugsweise durch untereinander parallel, insbesondere in Richtung auf die beiden an den zentralen Rückenabschnitt anschließenden Seitenabschnitte verlaufende Schlitze, ausgebildet sind.

Damit wird unter anderem die Nahtführung für den implantierenden Chirurgen erleichtert.

Bei besonders vorteilhaften Ausführungsform sind die beiden Seitenabschnitte exakt spiegelbildlich zueinander ausgebildet, was einerseits die Herstellung deutlich vereinfacht, andererseits auch für gleichmäßige Kraftverteilungen vom Implantat auf das von diesem umfasste Septum sorgt.

Bevorzugt sind Ausführungsformen des erfindungsgemäßen Implantats, bei welchen die Seitenabschnitte abgerundete Ecken aufweisen, um einerseits während der Implantation ein Hängenbleiben oder gar Aufspießen sicher zu verhindern, andererseits auch im implantierten Zustand eine Verletzungsgefahr auszuschließen.

Für einige Anwendungen kann es vorteilhaft sein, wenn das Implantat ganz oder teilweise aus Metall gefertigt ist. Als Material für chirurgische und orthopädische Implantate, die dauerhaft im Körper verbleiben sollen, sind Metalle und deren Legierungen prädestiniert, weil sie neben einer sehr guten Biokompatibilität hohe Dauerfestigkeit und Elastizität aufweisen. Trotz einer relativ geringen Dichte besitzen Implantate aus solchen Materialien wie Titan oder Titanverbindungen ausgezeichnete mechanische Eigenschaften mit einer langen Lebensdauer. Ebenfalls eine gute Eignung für die genannten Zwecke besitzt Edelstahl.

Bevorzugt sind Ausführungsformen, bei welchen das Implantat aus einem Werkstoff mit Memory-Effekt und/oder superelastischen Eigenschaften, vorzugsweise aus Nitinol, aufgebaut ist, so dass beispielsweise durch geeignete thermische Behandlung des Implantats optimale Federeigenschaften relativ zum Septum eingebracht werden können.

Für andere Anwendungen kann es vorteilhaft sein, wenn das Implantat ganz oder teilweise Kunststoff gefertigt ist, insbesondere aus Polymer-Material, vorzugsweise aus einem Material, welches eine ähnliche Flexibilität wie das menschliche Septum aufweist, aber über genügend Steifigkeiten verfügt, um eine gekrümmte Nasenscheidewand langfristig zu begradigen, und ohne eine Durchtrennung des Septums erforderlich zu machen um eine Schwächung herbeizuführen.

Zusätzlich zur guten Biokompatibilität des verwendeten Materials selbst kann das Implantat auch einen besonderen, körperverträglichen und/oder keimabweisenden Überzug aufweisen.

Zur Erzielung einer fein abgestimmten Form des Implantats kann dieses zweckmäßigerweise mittels 3D-Drucktechnik und/oder mittels Lasertechnik hergestellt sein.

Entsprechend den individuellen Anforderungen kann das erfindungsgemäße Implantat aber auch in spanender Technik, insbesondere mittels Frästechnik und/oder mittels Schleiftechnik, vorzugsweise Gleitschleifen, Magnetgleitschleifen oder Satelliten-Fliehkraftschleifen, und/oder mittels Poliertechnik, vorzugsweise Elektropolieren, oder in Ätztechnik hergestellt werden.

Bei weiteren vorteilhaften Ausführungsformen der Erfindung schließlich ist das Implantat im Spritzguss nach dem Micro Injection Moulding (= MIM) Verfahren gefertigt, welches beispielsweise aus der WO 00/06327 A2 an sich bekannt ist. Damit kann eine äußerst preisgünstige Herstellung auch sehr großer Stückzahlen bei gleichbleibender Maßgenauigkeit erreicht werden, während die herkömmlichen Implantate in der Regel wie etwa Schmuckwaren handangefertigt werden und daher einerseits relativ teuer in der Herstellung sind, andererseits in der Maßgenauigkeit individuell variieren können.

Weitere Merkmale und Vorteile der Erfindung ergeben sich aus der nachfolgenden detaillierten Beschreibung von Ausführungsbeispielen der Erfindung anhand der Figuren der Zeichnung, die erfindungswesentliche Einzelheiten zeigt, sowie aus den Ansprüchen.

### Detaillierte Beschreibung der Erfindung anhand der Zeichnung

In der schematischen Zeichnung sind Ausführungsbeispiele der Erfindung dargestellt, welche in der nachfolgenden Beschreibung näher erläutert werden.

Es zeigen:

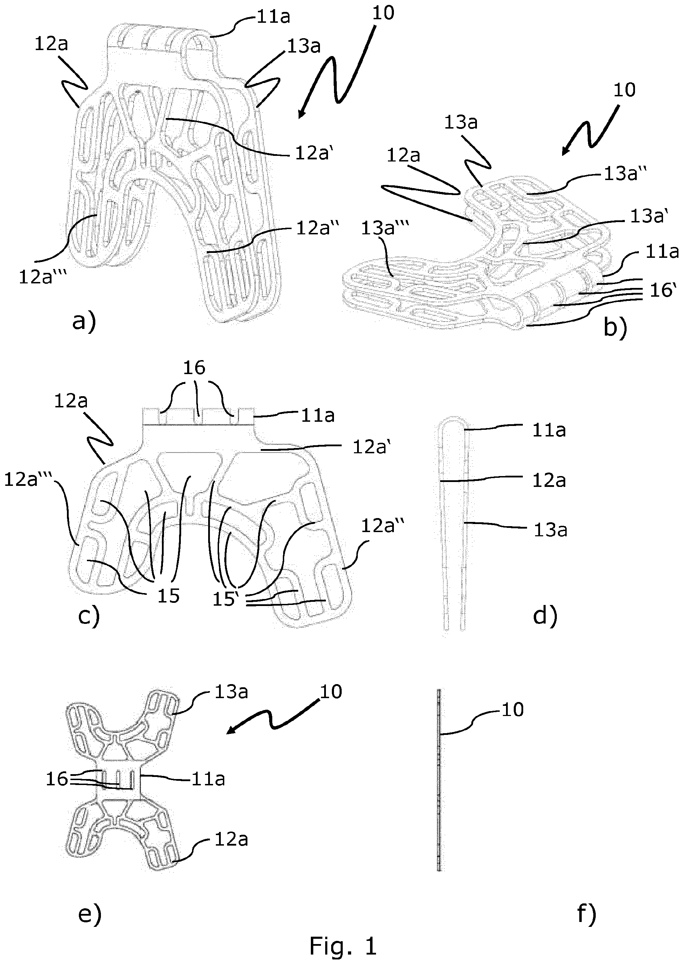

- Fign.1a-f: schematische, teils perspektivische Ansichten einer Ausführungsform des erfindungsgemäßen Implantats in kleiner S-Größe mit einer maximalen Gesamtausdehnung in einer Richtung parallel zur Achse des zentralen Rückenabschnitts zwischen 12 mm und 15 mm, wobei die Abbildungen das Implantat in unterschiedlichen Perspektiven darstellen, nämlich
a) räumlich mit Blickrichtung schräg von der Seite,
b) räumlich liegend,
c) eine Seitenansicht auf das Implantat mit Blickrichtung senkrecht zur Ebene eines Seitenabschnitts,
d) wie c), jedoch um 90° gedreht mit Blickrichtung parallel zur Ebene der Seitenabschnitte,
e) eine Draufsicht auf das noch flache, nicht-aufgefaltete Implantat mit den Seitenabschnitten in der Ebene des zentralen Rückenabschnitts liegend,
f) wie e), jedoch um 90° gedreht mit Blickrichtung parallel zur Ebene der Seitenabschnitte und des zentralen Rückenabschnitts;
- Fign. 2a-d: wie Fign. 1a-d, jedoch mit einer Ausführungsform des erfindungsgemäßen Implantats in mittlerer M-Größe mit einer maximalen Gesamtausdehnung in einer Richtung parallel zur Achse des zentralen Rückenabschnitts zwischen 16 mm und 20 mm; und
- Fign. 3a-d: wie Fign. 2a-d, jedoch mit einer Ausführungsform des erfindungsgemäßen Implantats in großer L-Größe mit einer maximalen Gesamtausdehnung in einer Richtung parallel zur Achse des zentralen Rückenabschnitts zwischen 20 mm und 25 mm.

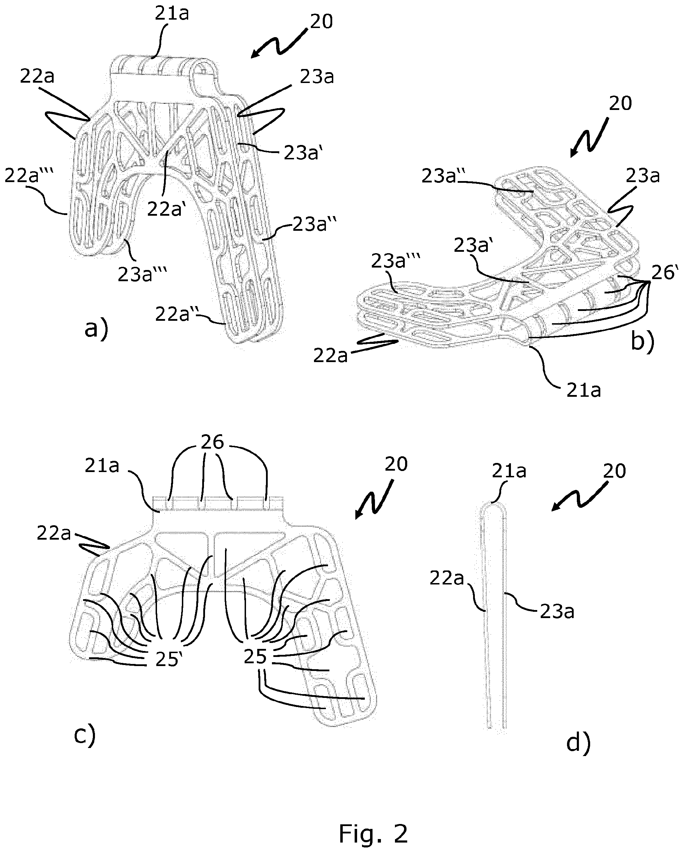

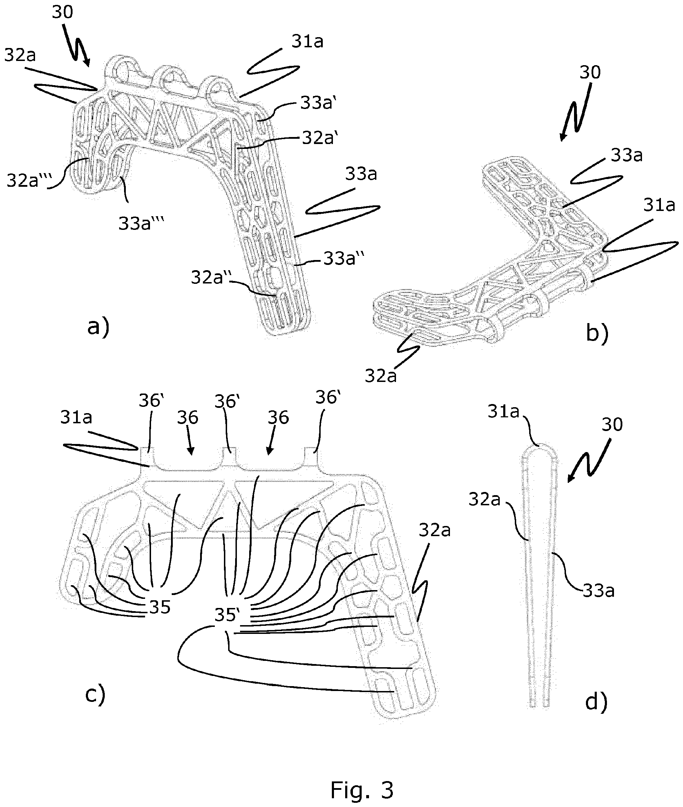

Das in den **Figuren 1a****-e, 2a-d und 3a-d** der Zeichnung gezeigte rhinologische **Implantat 10; 20; 30** zur Begradigung der Nasenscheidewand ist beiderseits des Septums der menschlichen Nase auf der jeweiligen Außenfläche des Septums in der jeweils linken und rechten Seite der Nasenhöhle befestigbar. Vor der Implantation wird das Implantat 10; 20; 30 aus einem zunächst flachen Zuschnitt in seine spätere, implantierfähige Raumform aufgefaltet.

Das Implantat 10; 20; 30 weist immer einen **zentralen Rückenabschnitt 11a; 21a; 31a** auf, der flach oder nur sehr leicht von einer Horizontalebene nach oben mit einem Spreizungswinkel ω > 100°, vorzugsweise ω > 160° abgewinkelt oder tonnenförmig mit einem Krümmungsradius r ≥ 0,5 mm von der Horizontalebene nach oben gekrümmt ist und im implantierten Zustand die freie Unterkante des Septums umgreift. Zwei **Seitenabschnitte 12a, 13a; 22a, 23a; 32a, 33a** sind beiderseits des zentralen Rückenabschnitts 11a; 21a; 31a vorgesehen, die symmetrisch zum zentralen Rückenabschnitt 11a; 21a; 31a unter einem Winkel ϕ von jeweils etwa 90° gegen den zentralen Rückenabschnitt 11a; 21a; 31a nach oben abgekantet verlaufen, und die im implantierten Zustand auf den beiden gegenüberliegenden Außenflächen des Septums vollflächig anliegen.

Bei sämtlichen in der Zeichnung dargestellten Ausführungsformen sind die Ecken der Seitenabschnitte 12a, 13a; 22a, 23a; 32a, 33a abgerundet.

Die beiden Seitenabschnitte 12a, 13a; 22a, 23a; 32a, 33a des Implantats 10; 20; 30 weisen jeweils einen **ersten Teilabschnitt 12a', 13a'; 22a', 23a'; 32a', 33a'** auf, der sich unmittelbar an den zentralen Rückenabschnitt 11a; 21a; 31a anschließt und im Wesentlichen parallel zu diesem verläuft. Zu ihren freien Enden hin weisen die beiden Seitenabschnitte 12a, 13a; 22a, 23a; 32a, 33a jeweils einen **zweiten Teilabschnitt 12a", 13a"; 22a", 23a"; 32a", 33a"** auf, der sich an den jeweiligen ersten Teilabschnitt 12a', 13a'; 22a', 23a'; 32a', 33a' anschließt und gegenüber diesem abgewinkelt verläuft. Insbesondere können die beiden Seitenabschnitte 12a, 13a; 22a, 23a; 32a, 33a des Implantats 10; 20; 30 eine winkelförmige Kontur, vorzugsweise eine V-förmige oder Bumerang-förmige Kontur aufweisen. Dadurch kann die mechanische Langzeit-Stabilität des Implantats erheblich erhöht und der flächige feste Sitz an den beiden Seitenflächen der Nasenscheidewand noch weiter verbessert werden.

Erfindungsgemäß zeichnet sich das Implantat 10; 20; 30 dadurch aus, dass sich an einem dem jeweiligen zweiten Teilabschnitt 12a", 13a"; 22a", 23a"; 32a", 33a" gegenüberliegenden Ende des jeweiligen ersten Teilabschnitts 12a', 13a'; 22a', 23a'; 32a', 33a'jeweils ein **dritter Teilabschnitt 12a‴, 13a‴; 22a‴, 23a‴; 32a‴, 33a‴** anschließt und gegenüber dem jeweiligen ersten Teilabschnitt 12a', 13a'; 22a', 23a'; 32a', 33a' ebenfalls abgewinkelt verläuft.

Der dritte Teilabschnitt 12a"', 13a‴; 22a"', 23a‴; 32a‴, 33a‴ weist eine größere vom ersten Teilabschnitt (12a', 13a'; 22a', 23a'; 32a', 33a' wegragende Längenausdehnung auf als der zweite Teilabschnitt 12a", 13a"; 22a", 23a"; 32a", 33a".

Bei den in der Zeichnung dargestellten Ausführungsformen der Erfindung sind die beiden Seitenabschnitte 12a, 13a; 22a, 23a; 32a, 33a des Implantats 10; 20; 30 jeweils mit **Perforationen 15; 25; 35** versehen sind, die zusammen einen größeren Flächeninhalt aufweisen als die die Perforationen 15; 25; 35 umgebenden, insbesondere Steg-förmig ausgebildeten, **festen Teilabschnitte 15'; 25'; 35'** der Seitenabschnitte 12a, 13a; 22a, 23a; 32a, 33a.

Die Perforationen 15; 25; 35 können -wie in den Figuren der Zeichnung gezeigt- Polyederförmig oder Wabenförmig, aber bei nicht eigens dargestellten Ausführungsform auch als kreisrunde Löcher oder auch als Langlöcher ausgebildet sein. Sie helfen, einerseits das Gewicht des Implantats zu verringern und andererseits den Anteil des körperfremden Materials im Körper eines Patienten so weit wie möglich zu reduzieren. Außerdem fördern die Perforationen 15; 25; 35 das Verwachsen des Implantats 10; 20; 30 mit dem umgebenden Gewebe.

Das Implantat 10; 20; 30 wird operativ durch eine sogenannte offene Rhinoplastik in die Nase eingebracht und auf dem Knorpel des Septums mittels einer Naht befestigt. Hierbei werden mehrere Einzelnähte durch die Perforationen 15; 25; 35 und das Septum angelegt und fixiert.

Auch der zentrale Rückenabschnitt 11a; 21a; 31a ist vorzugsweise mit **Perforationen 16; 26; 36** versehen, die zusammen einen kleineren Flächeninhalt aufweisen als die die Perforationen 16; 26; 36 umgebenden **festen Teilabschnitte 16'; 26'; 36'** des zentralen Rückenabschnitts 11a; 21a; 31a. Die Perforationen 16; 26; 36 des zentralen Rückenabschnitts 11a; 21a; 31a können Schlitzförmig, vorzugsweise durch untereinander parallel, insbesondere in Richtung auf die beiden an den zentralen Rückenabschnitt 11a; 21a; 31a anschließenden Seitenabschnitte 12a, 13a; 22a, 23a; 32a, 33a verlaufende Schlitze, ausgebildet sein.

Bei den in der Zeichnung dargestellten Ausführungsform des erfindungsgemäßen Implantats 10; 20; 30 sind die beiden Seitenabschnitte 12a, 13a; 22a, 23a; 32a, 33a jeweils exakt spiegelbildlich zueinander ausgebildet.

Das erfindungsgemäße Implantat 10; 20; 30 kann ganz oder teilweise aus Metall, insbesondere aus Titan, aus einer Titanlegierung oder aus Edelstahl und/oder aus einem Werkstoff mit Memory-Effekt und/oder superelastischen Eigenschaften, vorzugsweise aus Nitinol, gefertigt sein. Vorzugsweise wird es einen körperverträglichen Überzug aufweisen.

Das Implantat 10; 20; 30 kann aber auch ganz oder teilweise aus Kunststoff gefertigt sein, insbesondere aus Polymer-Material, vorzugsweise aus einem Material, welches eine ähnliche Flexibilität wie das menschliche Septum aufweist, aber über genügend Steifigkeiten verfügt, um eine gekrümmte Nasenscheidewand langfristig zu begradigen, und ohne eine Durchtrennung des Septums erforderlich zu machen um eine Schwächung herbeizuführen.

Das Implantat 10; 20; 30 kann mittels 3D-Drucktechnik und/oder mittels Lasertechnik hergestellt werden. Möglich ist auch, das Implantat 10; 20; 30 in spanender Technik, insbesondere mittels Frästechnik und/oder mittels Schleiftechnik, vorzugsweise Gleitschleifen, Magnetgleitschleifen oder Satellitenfliehkraftschleifen, und/oder mittels Poliertechnik, vorzugsweise Elektropolieren, oder in Ätztechnik herzustellen und zu bearbeiten. Außerdem kann das Implantat 10; 20; 30 beispielsweise auch im Spritzguss nach dem Micro Injection Moulding (=MIM) Verfahren gefertigt werden.

Zweck des erfindungsgemäßen Implantats ist eine Stabilisierung, Schienung und Begradigung der vorderen (anterioren) Nasenscheidewand bis zum hinteren (dorsalen) Ende des Implantats. Indikationen können zum Beispiel Septums-Deviationen des anterioren Septums bis zu ca. 15 mm in Folge von Traumata, kongenitaler Deviation usw. sein.

In einem Labortest in der Anatomie an der Universität Tübingen wurde an einem Körperspender getestet, ob die erfindungsgemäßen Implantate bei einer äußeren Krafteinwirkung von 20 N im implantierten Zustand bleibende deformiert werden oder stabil bleiben. Es wurden verschiedene Design-Varianten und Größen des erfindungsgemäßen Septum-Implantats getestet. Varianten, die diesen Test ohne bleibende Deformierung überstanden haben, wozu auch die hier gezeigten Ausführungsformen gehörten, wurden als akzeptiert betrachtet. Die drei aktuellen Größen "S", "M" und "L" aus Reintitan, Medical Grade 4, mit einer Materialdicke von 0,25 mm haben diesen Test bestanden. Für eine noch höhere Sicherheit in der Stabilität wurde eine Materialdicke von 0,4 mm gewählt. Die maximale Gesamtausdehnung in einer Richtung parallel zur Achse des zentralen Rückenabschnitts beträgt derzeit bei Implantaten der Größe "L" 22,6 mm, bei der Größe "M" 17,8 mm.

### Bezuaszeichenliste:

- 10; 20; 30: rhinologisches Implantat
- 11a; 21a; 31a: zentraler Rückenabschnitt
- 12a, 13a; 22a, 23a; 32a, 33a: gegenüberliegende Seitenabschnitte
- 12a', 13a'; 22a', 23a'; 32a', 33a': erste Teilabschnitte
- 12a", 13a"; 22a", 23a"; 32a", 33a": zweite Teilabschnitte
- 12a'", 13a‴; 22a‴, 23a‴; 32a‴, 33a‴: dritte Teilabschnitte
- 15; 25; 35: Perforationen der Seitenabschnitte
- 15'; 25'; 35': feste Teilabschnitte der Seitenabschnitte
- 16; 26; 36: Perforationen des Rückenabschnitts
- 16'; 26'; 36': feste Teilabschnitte des Rückenabschnitts

### Referenzliste:

Für die Beurteilung der Patentfähigkeit in Betracht gezogene Publikationen:
[1] DE 10 2012 107 123 B4 ≈ EP 2 692 313 B1 ≈ US 9,895,252 B2
[2] Internetseite vom 09.08.2022 https://www.bess.eu/de/rhinoloqie/septumschienen/
[3] US 2012/0078367 A1
[4] WO 2008/153263 A1
[5] US 6,322,590 B1
[6] EP 1 475 056 B1
[7] DE 2006 023 058 B3
[8] HNO. 1999 Jun; 47(6):546-50
[9] CN 21 538 4901 U
[10] US 11,241,306 B2

## Patentansprüche

1. Rhinologisches Implantat (10; 20; 30) zur Begradigung der Nasenscheidewand, das beiderseits des Septums der menschlichen Nase auf der jeweiligen Außenfläche des Septums in der jeweils linken und rechten Seite der Nasenhöhle befestigbar ist, wobei das Implantat (10; 20; 30) einen zentralen Rückenabschnitt (11a; 21a; 31a) aufweist, der flach oder nur sehr leicht von einer Horizontalebene nach oben mit einem Spreizungswinkel ω > 100° abgewinkelt oder tonnenförmig mit einem Krümmungsradius r ≥ 0,5mm von der Horizontalebene nach oben gekrümmt ist und im implantierten Zustand die freie Unterkante des Septums umgreift, wobei zwei Seitenabschnitte (12a, 13a; 22a, 23a; 32a, 33a) des Implantats (10; 20; 30) beiderseits des zentralen Rückenabschnitts (11a; 21a; 31a) vorgesehen sind, die symmetrisch zum zentralen Rückenabschnitt (11a; 21a; 31a) unter einem Winkel ϕ von jeweils etwa 90° gegen den zentralen Rückenabschnitt (11a; 21a; 31a) nach oben abgekantet verlaufen, und die im implantierten Zustand auf den beiden gegenüberliegenden Außenflächen des Septums vollflächig anliegen, wobei das Implantat (10; 20; 30) vor der Implantation aus einem zunächst flachen Zuschnitt in seine spätere, implantierfähige Raumform aufgefaltet ist, wobei die beiden Seitenabschnitte (12a, 13a; 22a, 23a; 32a, 33a) des Implantats (10; 20; 30) jeweils einen ersten Teilabschnitt (12a', 13a'; 22a', 23a'; 32a', 33a') aufweisen, der sich unmittelbar an den zentralen Rückenabschnitt (11a; 21a; 31a) anschließt und im Wesentlichen parallel zu diesem verläuft, und wobei die beiden Seitenabschnitte (12a, 13a; 22a, 23a; 32a, 33a) jeweils einen zweiten Teilabschnitt (12a", 13a"; 22a", 23a"; 32a", 33a") aufweisen, der sich an den jeweiligen ersten Teilabschnitt (12a', 13a'; 22a', 23a'; 32a', 33a') anschließt und gegenüber diesem abgewinkelt verläuft,
**dadurch gekennzeichnet,**
**dass** sich an einem dem jeweiligen zweiten Teilabschnitt (12a",13a"; 22a", 23a"; 32a", 33a") gegenüberliegenden Ende des jeweiligen ersten Teilabschnitts (12a', 13a'; 22a', 23a'; 32a', 33a') jeweils ein dritter Teilabschnitt (12a‴, 13a‴; 22a‴, 23a‴, 32a‴, 33a‴) anschließt und gegenüber dem jeweiligen ersten Teilabschnitt (12a', 13a'; 22a', 23a'; 32a', 33a') ebenfalls abgewinkelt verläuft.

2. Implantat nach Anspruch 1, **dadurch gekennzeichnet, dass** der dritte Teilabschnitt (12a‴, 13a‴; 22a‴, 23a‴; 32a‴ ,33a‴) eine größere vom ersten Teilabschnitt (12a', 13a'; 22a', 23a'; 32a', 33a') wegragende Längenausdehnung aufweist als der zweite Teilabschnitt (12a", 13a"; 22a", 23a"; 32a", 33a").

3. Implantat nach Anspruch 1 oder 2, **dadurch gekennzeichnet, dass** die beiden Seitenabschnitte (12a, 13a; 22a, 23a; 32a, 33a) des Implantats (10; 20; 30) jeweils eine Bumerang-förmige Kontur aufweisen.

4. Implantat nach einem der vorhergehenden Ansprüche, **dadurch gekennzeichnet, dass** die beiden Seitenabschnitte (12a, 13a; 22a, 23a; 32a, 33a) des Implantats (10; 20; 30) jeweils mit Perforationen (15; 25; 35) versehen sind, und dass die Perforationen (15; 25; 35) zusammen einen größeren Flächeninhalt aufweisen als die die Perforationen (15; 25; 35) umgebenden, insbesondere Steg-förmig ausgebildeten, festen Teilabschnitte (15'; 25'; 35') der Seitenabschnitte (12a, 13a; 22a, 23a; 32a, 33a).

5. Implantat nach Anspruch 4, **dadurch gekennzeichnet, dass** zumindest ein Teil der Perforationen (15; 25; 35) Polyederförmig oder Wabenförmig ausgebildet ist.

6. Implantat nach einem der vorhergehenden Ansprüche, **dadurch gekennzeichnet, dass** der zentrale Rückenabschnitt (11a; 21a; 31a) mit Perforationen (16; 26; 36) versehen ist, und dass die Perforationen (16; 26; 36) des zentralen Rückenabschnitts (11a; 21a; 31a) zusammen einen kleineren Flächeninhalt aufweisen als die die Perforationen (16; 26; 36) umgebenden festen Teilabschnitte (16'; 26'; 36') des zentralen Rückenabschnitts (11a; 21a; 31a).

7. Implantat nach Anspruch 6, **dadurch gekennzeichnet, dass** die Perforationen (16; 26; 36) Schlitzförmig, vorzugsweise durch untereinander parallel, insbesondere in Richtung auf die beiden an den zentralen Rückenabschnitt (11a; 21a; 31a) anschließenden Seitenabschnitte (12a, 13a; 22a, 23a; 32a, 33a) verlaufende Schlitze, ausgebildet sind.

8. Implantat nach einem der vorhergehenden Ansprüche, **dadurch gekennzeichnet, dass** die beiden Seitenabschnitte (12a, 13a; 22a, 23a; 32a, 33a) exakt spiegelbildlich zueinander ausgebildet sind.

9. Implantat nach einem der vorhergehenden Ansprüche, **dadurch gekennzeichnet, dass** sämtliche Ecken der Seitenabschnitte (12a, 13a; 22a, 23a; 32a, 33a) abgerundet sind.

10. Implantat nach einem der vorhergehenden Ansprüche, **dadurch gekennzeichnet, dass** das Implantat (10; 20; 30) aus einem Werkstoff mit Memory-Effekt und/oder superelastischen Eigenschaften, vorzugsweise aus Nitinol, aufgebaut ist.

11. Implantat nach einem der vorhergehenden Ansprüche, **dadurch gekennzeichnet, dass** das Implantat (10; 20; 30) ganz oder teilweise aus Kunststoff gefertigt ist, insbesondere aus Polymer-Material, vorzugsweise aus einem Material, welches eine ähnliche Flexibilität wie das menschliche Septum aufweist, aber über genügend Steifigkeiten verfügt, um eine gekrümmte Nasenscheidewand langfristig zu begradigen, und ohne eine Durchtrennung des Septums erforderlich zu machen um eine Schwächung herbeizuführen.

12. Implantat nach einem der vorhergehenden Ansprüche, **dadurch gekennzeichnet, dass** das Implantat (10; 20; 30) einen körperverträglichen Überzug aufweist.

13. Implantat nach einem der Ansprüche 1 bis 12, **dadurch gekennzeichnet, dass** das Implantat (10; 20; 30) mittels 3D-Drucktechnik und/oder mittels Lasertechnik hergestellt ist.

14. Implantat nach einem der Ansprüche 1 bis 12, **dadurch gekennzeichnet, dass** das Implantat (10; 20; 30) in spanender Technik, insbesondere mittels Frästechnik und/oder mittels Schleiftechnik, vorzugsweise Gleitschleifen, Magnetgleitschleifen oder Satellitenfliehkraftschleifen, und/oder mittels Poliertechnik, vorzugsweise Elektropolieren, oder in Ätztechnik hergestellt ist.

15. Implantat nach einem der Ansprüche 1 bis 12, **dadurch gekennzeichnet, dass** das Implantat (10; 20; 30) im Spritzguss nach dem Micro Injection Moulding (= MIM) Verfahren gefertigt ist.

## Claims

1. Rhinological implant (10; 20; 30) for straightening the nasal septum, which implant can be fixed on both sides of the septum of the human nose on the relevant outer surface of the septum in each left and right side of the nasal cavity, wherein the implant (10; 20; 30) has a central tergal portion (11a; 21a; 31a) which is flat or only very slightly angled upward from a horizontal plane at a spread angle ω > 100°, or is curved upward from the horizontal plane in a barrel-shaped manner with a radius of curvature r ≥ 0.5 mm, and, in the implanted state, encompasses the free lower edge of the septum, wherein two side portions (12a, 13a; 22a, 23a; 32a, 33a) of the implant (10; 20; 30) are arranged on either side of the central tergal portion (11a; 21a; 31a), which side portions extend so as to be bent upward, symmetrically to the central tergal portion (11a; 21a; 31a), at an angle ϕ of in each case approximately 90° with respect to the central tergal portion (11a; 21a; 31a), and, in the implanted state, abut the entire surface of the two opposite outer surfaces of the septum, wherein, before implantation, the implant (10; 20; 30) is folded from an initially flat blank into its subsequent, implantable three-dimensional shape, wherein the two side portions (12a, 13a; 22a, 23a; 32a, 33a) of the implant (10; 20; 30) each have a first partial portion (12a', 13a'; 22a', 23a'; 32a', 33a*) which connects directly to the central tergal portion (11a; 21a; 31a) and extends substantially in parallel therewith, and wherein the two side portions (12a, 13a; 22a, 23a; 32a, 33a) each have a second partial portion (12a", 13a"; 22a", 23a"; 32a", 33a") which connects to the relevant first partial portion (12a', 13a'; 22a', 23a'; 32a', 33a') and extends at an angle relative thereto,
**characterized in that**
a third partial portion (12a‴, 13a‴; 22a‴, 23a‴; 32a‴, 33a‴) is connected to an end of the relevant first partial portion (12a', 13a'; 22a', 23a'; 32a', 33a*) opposite the relevant second partial portion (12a", 13a"; 22a", 23a"; 32a", 33a") and also extends at an angle relative to the relevant first partial portion (12a', 13a'; 22a', 23a'; 32a', 33a').

2. Implant according to claim 1, **characterized in that** the third partial portion (12a‴, 13a‴; 22a‴, 23a‴; 32a‴, 33a‴) has a larger longitudinal extension projecting away from the first partial portion (12a', 13a'; 22a', 23a'; 32a', 33a') than the second partial portion (12a", 13a"; 22a", 23a"; 32a", 33a").

3. Implant according to claim 1 or 2, **characterized in that** the two side portions (12a, 13a; 22a, 23a; 32a, 33a) of the implant (10; 20; 30) each have a boomerang-shaped contour.

4. Implant according to any of the preceding claims, **characterized in that** the two side portions (12a, 13a; 22a, 23a; 32a, 33a) of the implant (10; 20; 30) are each provided with perforations (15; 25; 35), and **in that** the perforations (15; 25; 35) together have a larger surface area than the fixed partial portions (15'; 25'; 35*), in particular in the form of connecting portions, surrounding the perforations (15; 25; 35), of the side portions (12a, 13a; 22a, 23a; 32a, 33a).

5. Implant according to claim 4, **characterized in that** at least some of the perforations (15; 25; 35) have the shape of a polyhedron or honeycomb.

6. Implant according to any of the preceding claims, **characterized in that** the central tergal portion (11a; 21a; 31a) is provided with perforations (16; 26; 36), and **in that** the perforations (16; 26; 36) of the central tergal portion (11a; 21a; 31a) together have a smaller surface area than the fixed partial portions (16'; 26'; 36') of the central tergal portion (11a; 21a; 31a) which surround the perforations (16; 26; 36).

7. Implant according to claim 6, **characterized in that** the perforations (16; 26; 36) are slot shaped, preferably formed by slots extending in parallel with one another, in particular in the direction of the two side portions (12a, 13a; 22a, 23a; 32a, 33a) connecting to the central tergal portion (11a; 21a; 31a).

8. Implant according to any of the preceding claims, **characterized in that** the two side portions (12a, 13a; 22a, 23a; 32a, 33a) are designed to be exact mirror images of each other.

9. Implant according to any of the preceding claims, **characterized in that** all corners of the side portions (12a, 13a; 22a, 23a; 32a, 33a) are rounded.

10. Implant according to any of the preceding claims, **characterized in that** the implant (10; 20; 30) is made of a material having a memory effect and/or superelastic properties, preferably of nitinol.

11. Implant according to any of the preceding claims, **characterized in that** the implant (10; 20; 30) is made entirely or partially of plastics material, in particular of polymer material, preferably of a material which has a flexibility similar to the human septum, but has sufficient stiffnesses for straightening a curved nasal septum in the long term, and without requiring severing of the septum in order to cause weakening.

12. Implant according to any of the preceding claims, **characterized in that** the implant (10; 20; 30) has a biocompatible coating.

13. Implant according to any of claims 1 to 12, **characterized in that** the implant (10; 20; 30) is produced by 3D printing and/or by means of laser technology.

14. Implant according to any of claims 1 to 12, **characterized in that** the implant (10; 20; 30) is produced by machining, in particular by milling and/or by grinding, preferably sliding grinding, magnetic sliding grinding or satellite centrifugal grinding, and/or by polishing, preferably electropolishing, or by etching.

15. Implant according to any of claims 1 to 12, **characterized in that** the implant (10; 20; 30) is manufactured by injection molding according to the micro injection-molding (MIM) method.

## Revendications

1. Implant rhinologique (10 ; 20 ; 30) destiné à redresser la cloison nasale, qui peut être fixé de part et d'autre du septum du nez humain sur la surface externe respective du septum dans les côtés gauche et droit respectifs de la cavité nasale, l'implant (10 ; 20 ; 30) présentant une section dorsale centrale (11a ; 21a ; 31a) qui est plate ou seulement très légèrement inclinée vers le haut à partir d'un plan horizontal sous un angle d'écartement w > 100° ou qui est courbée vers le haut en forme de tonneau avec un rayon de courbure r ≥ 0,5 mm à partir du plan horizontal et qui entoure, à l'état implanté, le bord inférieur libre du septum, deux sections latérales (12a, 13a ; 22a, 23a ; 32a, 33a) de l'implant (10 ; 20 ; 30) étant prévues de part et d'autre de la section dorsale centrale (11a ; 21a ; 31a), lesquelles sections s'étendent de manière symétrique par rapport à la section dorsale centrale (11a ; 21a ; 31a) en étant repliées vers le haut sous un angle ϕ d'environ 90° chacune contre la section dorsale centrale (11a ; 21a ; 31a) et, à l'état implanté, s'appliquent sur toute leur surface sur les deux surfaces externes opposées du septum, l'implant (10 ; 20 ; 30) étant déplié avant l'implantation à partir d'une découpe d'abord plate dans sa forme spatiale ultérieure implantable, les deux sections latérales (12a, 13a ; 22a, 23a ; 32a, 33a) de l'implant (10 ; 20 ; 30) présentant chacune une première section partielle (12a', 13a'; 22a', 23a'; 32a', 33a'), qui se raccorde directement à la section dorsale centrale (11a ; 21a ; 31a) et s'étend sensiblement parallèlement à celle-ci, et les deux sections latérales (12a, 13a ; 22a, 23a ; 32a, 33a) présentant chacune une deuxième section partielle (12a", 13a" ; 22a", 23a" ; 32a", 33a") qui se raccorde à la première section partielle (12a', 13a'; 22a', 23a'; 32a', 33a') respective et s'étend en formant un angle par rapport à celle-ci,
**caractérisé en ce qu'**
en une extrémité opposée à la deuxième section partielle (12a", 13a" ; 22a", 23a"; 32a", 33a") respective de la première section partielle (12a', 13a'; 22a', 23a' ; 32a', 33a') respective, une troisième section partielle (12a‴, 13a‴ ; 22a‴, 23a‴ ; 32a‴, 33a‴) se raccorde respectivement et s'étend également en formant un angle par rapport à la première section partielle (12a', 13a' ; 22a', 23a' ; 32a', 33a') respective.

2. Implant selon la revendication 1, **caractérisé en ce que** la troisième section partielle (12a‴, 13a‴; 22a‴, 23a‴ ; 32a‴, 33a‴) présente une extension longitudinale s'écartant de la première section partielle (12a', 13a' ; 22a', 23a' ; 32a', 33a") plus grande que celle de la deuxième section partielle (12a", 13a" ; 22a", 23a" ; 32a", 33a").

3. Implant selon la revendication 1 ou 2, **caractérisé en ce que** les deux sections latérales (12a, 13a ; 22a, 23a ; 32a, 33a) de l'implant (10 ; 20 ; 30) présentent chacune un contour en forme de boomerang.

4. Implant selon l'une des revendications précédentes,
**caractérisé en ce que** les deux sections latérales (12a, 13a ; 22a, 23a ; 32a, 33a) de l'implant (10 ; 20 ; 30) sont chacune pourvues de perforations (15 ; 25 ; 35) et **en ce que** les perforations (15 ; 25 ; 35) présentent ensemble une superficie plus grande que celle des sections partielles fixes (15'; 25'; 35*) des sections latérales (12a, 13a ; 22a, 23a ; 32a, 33a) entourant les perforations (15 ; 25 ; 35), en particulier en forme d'âme.

5. Implant selon la revendication 4, **caractérisé en ce qu'**au moins une partie des perforations (15 ; 25 ; 35) est de forme polyédrique ou en nid d'abeilles.

6. Implant selon l'une des revendications précédentes,
**caractérisé en ce que** la section dorsale centrale (11a ; 21a ; 31a) est pourvue de perforations (16 ; 26 ; 36) et **en ce que** les perforations (16 ; 26 ; 36) de la section dorsale centrale (11a ; 21a ; 31a) présentent ensemble une superficie plus petite que celle des sections partielles fixes (16' ; 26' ; 36') de la section dorsale centrale (11a ; 21a ; 31a) entourant les perforations (16 ; 26 ; 36).

7. Implant selon la revendication 6, **caractérisé en ce que** les
perforations (16 ; 26 ; 36) sont réalisées en forme de fentes, de préférence par des fentes s'étendant parallèlement entre elles, en particulier en direction des deux sections latérales (12a, 13a ; 22a, 23a ; 32a, 33a) se raccordant à la section dorsale centrale (11a ; 21a ; 31a).

8. Implant selon l'une des revendications précédentes,
**caractérisé en ce que** les deux sections latérales (12a, 13a ; 22a, 23a ; 32a, 33a) sont réalisées de manière exactement symétrique l'une par rapport à l'autre.

9. Implant selon l'une des revendications précédentes,
**caractérisé en ce que** tous les coins des sections latérales (12a, 13a ; 22a, 23a ; 32a, 33a) sont arrondis.

10. Implant selon l'une des revendications précédentes,
**caractérisé en ce que** l'implant (10 ; 20 ; 30) est constitué d'un matériau doté d'un effet de mémoire et/ou de propriétés superélastiques, de préférence en Nitinol.

11. Implant selon l'une des revendications précédentes,
**caractérisé en ce que** l'implant (10 ; 20 ; 30) est fabriqué en tout ou en partie en matériau synthétique, notamment en matériau polymère, de préférence en un matériau présentant une flexibilité similaire à celle du septum humain, mais disposant de rigidités suffisantes pour redresser à long terme une cloison nasale courbée et sans nécessiter de sectionner le septum pour provoquer un affaiblissement.

12. Implant selon l'une des revendications précédentes,
**caractérisé en ce que** l'implant (10 ; 20 ; 30) présente un revêtement toléré par le corps.

13. Implant selon l'une des revendications 1 à 12, **caractérisé en ce que** l'implant (10 ; 20 ; 30) est fabriqué au moyen de la technique d'impression 3D et/ou de la technique au laser.

14. Implant selon l'une des revendications 1 à 12, **caractérisé en ce que** l'implant (10 ; 20 ; 30) est fabriqué par une technique d'enlèvement de copeaux, en particulier au moyen d'une technique de fraisage et/ou d'une technique de meulage, de préférence le meulage vibrant, le meulage vibrant magnétique ou le meulage à force centrifuge par satellite, et/ou au moyen d'une technique de polissage, de préférence l'électropolissage, ou par une technique de gravure.

15. Implant selon l'une des revendications 1 à 12, **caractérisé en ce que** l'implant (10 ; 20 ; 30) est fabriqué par moulage par injection selon le procédé de moulage par micro-injection (= MIM).
